# EUROPEAN PATENT APPLICATION

(11) **EP 4 378 470 A1**
(43) Date of publication of application: **05.06.2024**
(21) Application number: 22815452.2
(22) Date of filing: 30.05.2022
(51) Int. Cl.: A61K 36/899, A61K 36/23, A61K 125/00, A61P 19/10, A61P 19/02, A61K 9/70

(54) **TOPICAL NATURAL EXTRACT FOR SKIN FOR TREATMENT OF JOINT DEGENERATION AND OSTEOPOROSIS AND METHOD**

(30) Priority: 30.05.2021 US 202163195054 P
(71) Applicant: Liu, Yuk Kwan, New Territories Hong Kong (HK)
(72) Inventor: Liu, Yuk Kwan, New Territories Hong Kong (HK)
(74) Representative: Forresters IP LLP
(86) International application number: PCT/IB2022/055058
(87) International publication number: WO 2022/254316

(57) **Abstract**

The present invention is a natural plant extract for external treatment, alleviation, and improvement of joint degeneration and osteoporosis, recovery of mobility and elimination of pain. In addition, the use of natural plant extracts applied on the skin near or on the joint may relieve or eliminate pain or restore mobility to joints of patients with joint degeneration and osteoporosis.

## Description

### Background:

Main direction of existing practices of the field of drug research has been single chemical composition. In this approach, it is easier to focus on the mechanism of action of the single chemical composition. On the other hand, multi-substance or multi-chemical composition of the drug (such as compound ingredient drugs and Chinese medicine) are more complex. Due to the raw materials, molecular weight, molecular structure, boiling point, polarization, saponification value, iodine value, disintegration degree, solubility and volatility and other physical and biochemical indicators are different for each of the ingredients, the chemical complexity and variability is exponentially more difficult to understand or evaluate. In a pharmaceutical research, the more material and compound and the number of chemical component, the more complex the molecular physical and biochemical reactions are between them. As such, it is more difficult to predict and master the results, so that the difficulty of the research of composite drugs is much higher than that of drugs of a single chemical composition.

In order to search for diversity of biological functionality and to identify functional products' developments and research to promote overall health and combat aging, having a variety of natural substance compound is one of the directions in the drug research field and functional product technology breakthrough. While a variety of natural substances and molecular compounds due to the number of complexes and molecular components intersecting and biochemical reactions are complex and the compound process will produce a lot of uncertainty, at the same time of generating uncertainty, the probability of producing medicinal therapeutic functions also increases and heightens. In addition, the newly produced functional effects are often completely impossible to produce or do not exist in the single chemical composition or a single substance. Therefore, the use of natural substances containing a variety of chemical ingredients or the use of composite ways to find new therapeutic disease functions include potential and opportunity in the pharmaceutical research and functional product development.

### Summary:

One embodiment of the invention comprises a skin or topical product made mainly of natural extracts that may treat and improve joint degeneration and osteoporosis, may help restore mobility and may eliminate pain. At the same time, in another embodiment, through the topical natural extracts, may improve joint degeneration and osteoporosis, may restore mobility and may eliminate pain. In one embodiment, for female users, but not limited to female users, rubbing products embodying aspects of the invention of the natural extracts on the joint area on the skin, not only may improve the symptoms of degenerative arthritis and osteoporosis of the joint, but also may relieve or mediate the pain and symptoms caused by menopause in women, and restore mobility. Further in one embodiment, the natural extracts of the invention comprise an active ingredient of plants or grass in the species gramineae or poaceae, such as the vetiveria zizanioides or vetiver (chrysopogon zizanioides) or Andropogon zizanioides (hereinafter collectively referred to as vetiver). In addition, in one embodiment, the natural extracts of the invention comprise plants or vegetation in the species apiaceae, such as carrots or daucus carota. In one embodiment, carrot seeds may be used to obtain extracts from carrots or daucus carota.

In the embodiment of the invention, a product for external application on the skin may be absorbed through the skin to increase efficiency and effectiveness of aspects of the invention by avoiding or alleviating conditions such as the disruption of digestive process that is typical to ingestion application (e.g., oral medication) or the first-pass effect of the liver. In one aspect, the external use or application of embodiments of the invention may treat abnormal hormones, restore estradiol to normal, and restore estradiol to pre-symptomatic level before osteoporosis, prevent bone calcium loss and transfer thereof to blood and urine, improve bone strength, and prevent fractures and treat osteoporosis

### Brief Description of The Drawings

Those skilled in the art may appreciate that the elements in the artwork are used to illustrate simplicity and clarity, so not all coherence and selection are expressed. For example, common but well-known elements that are useful or necessary in commercially viable embodiments may generally not be described in order to have fewer obstacles to these various embodiments of the present disclosure. It will be further appreciated that, while those skilled in the art may appreciate, in fact, this particularity in terms of sequence is not required. It is also understandable that the terms and phrases used in the present case may define their respective areas of investigation and research, unless otherwise specified herein have specific meanings.

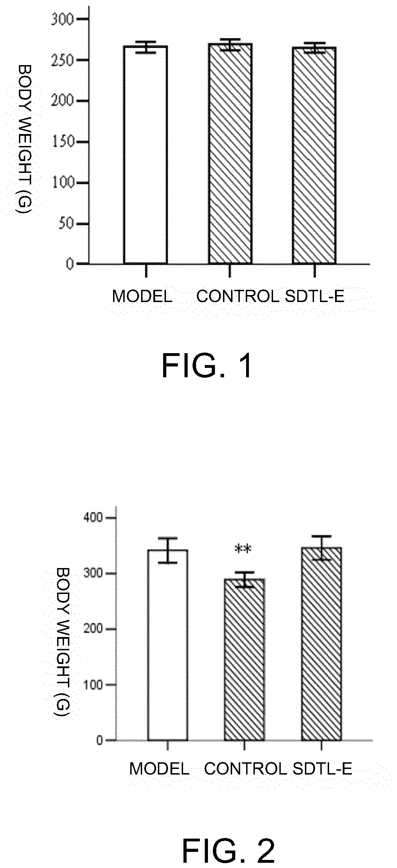

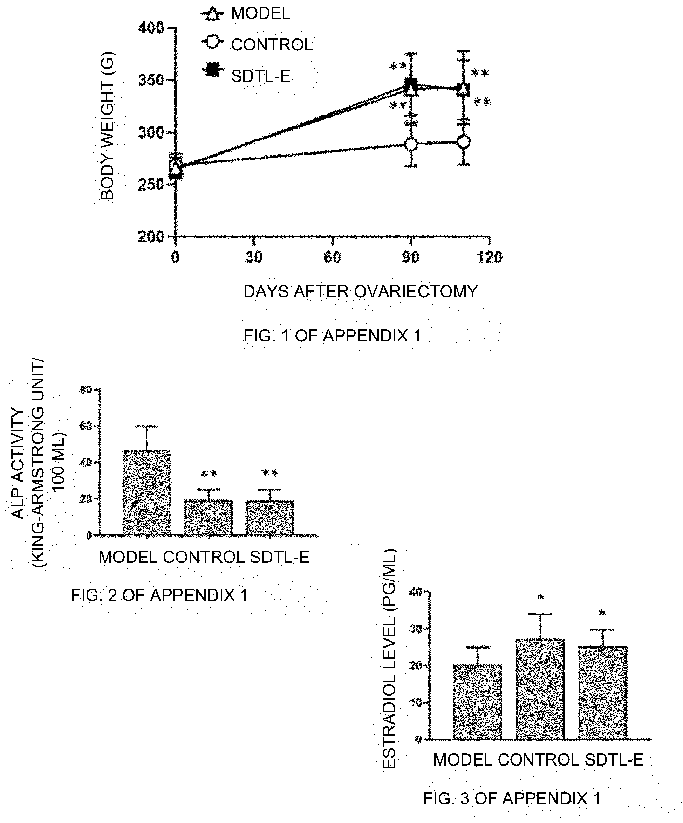

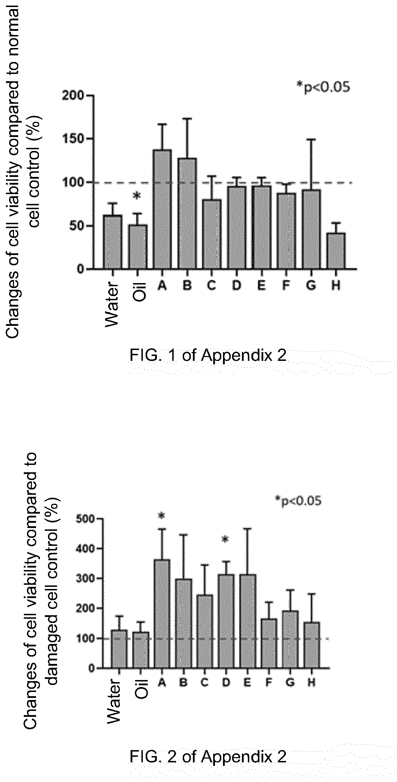

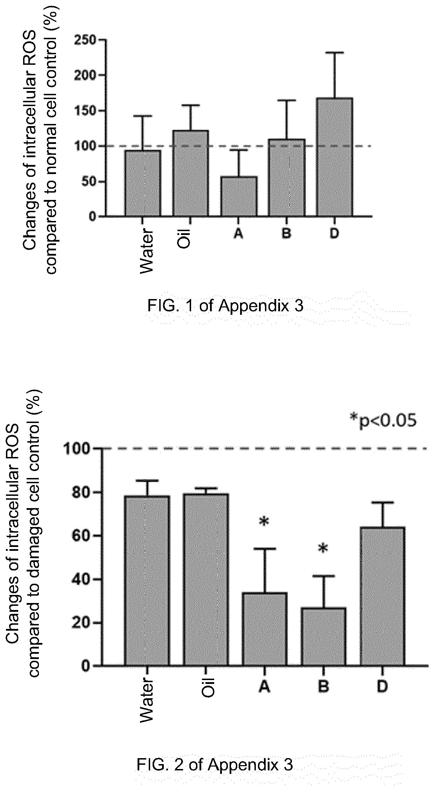

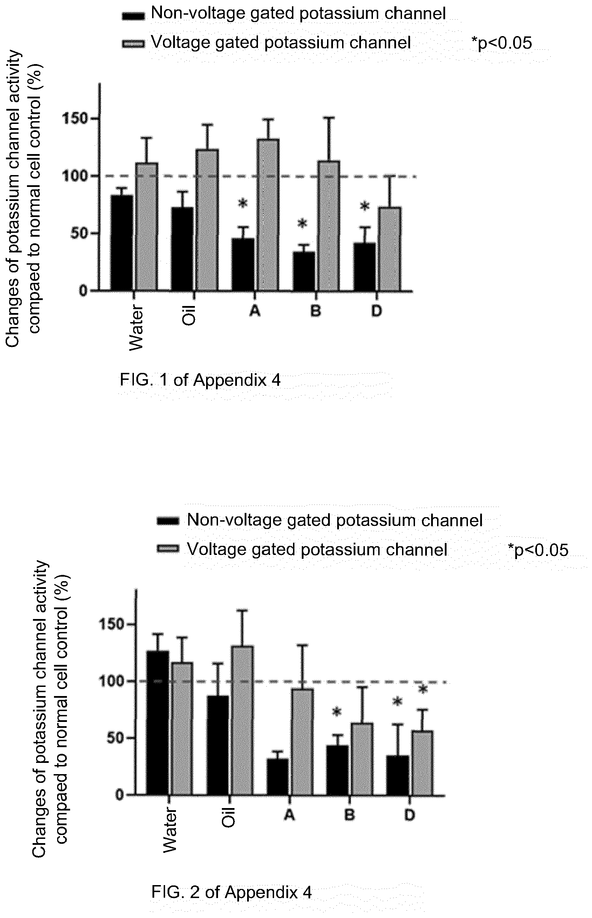

FIG. 1 shows the weight of mice before drug treatment in animal experiments according to one embodiment.
FIG. 2 shows the weight of mice on day 1 after drug treatment in animal experiments according to one embodiment.

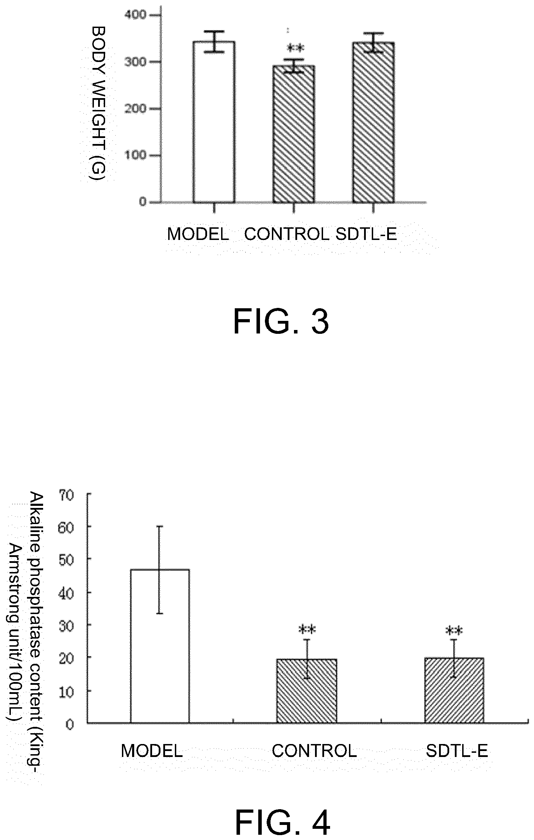

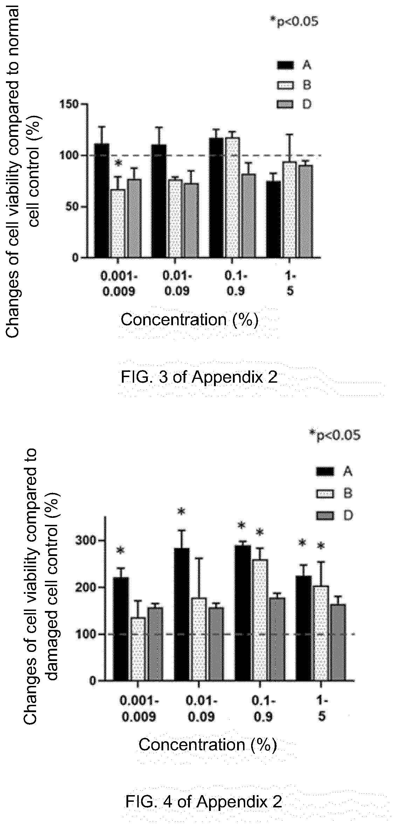

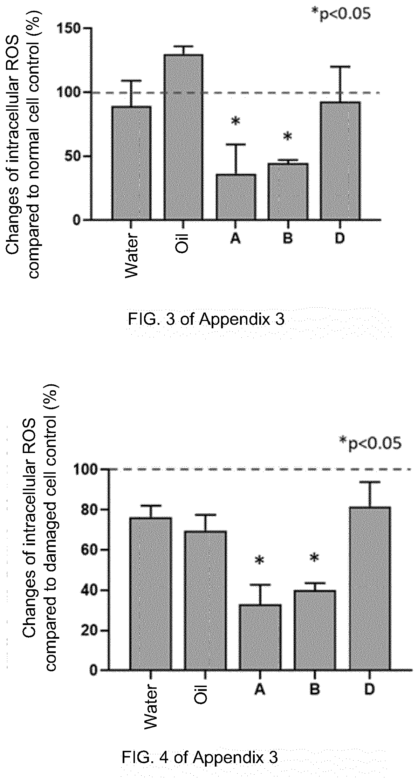

FIG. 3 shows the weight of mice 20 days after drug treatment in animal experiments according to one embodiment.

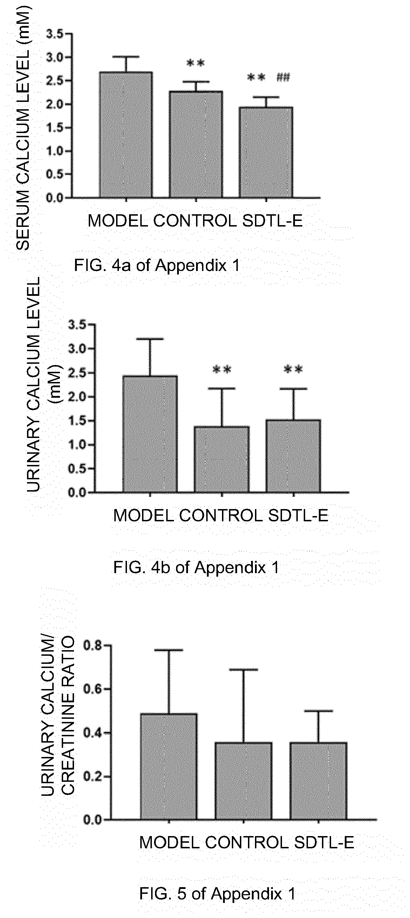

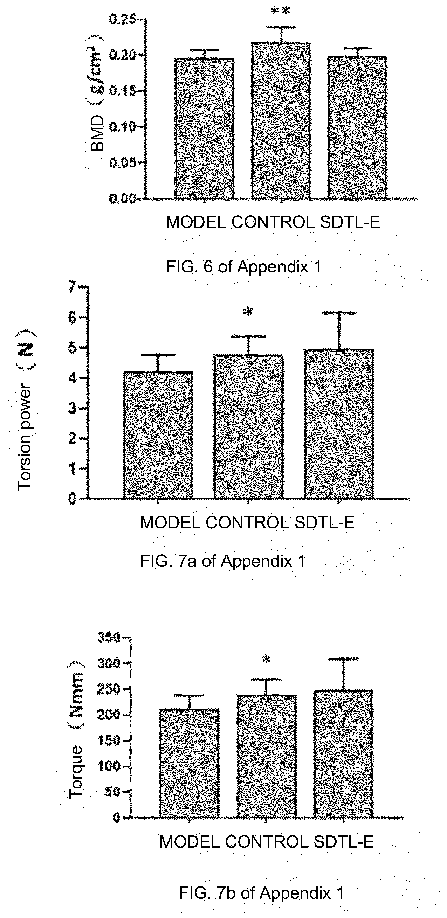

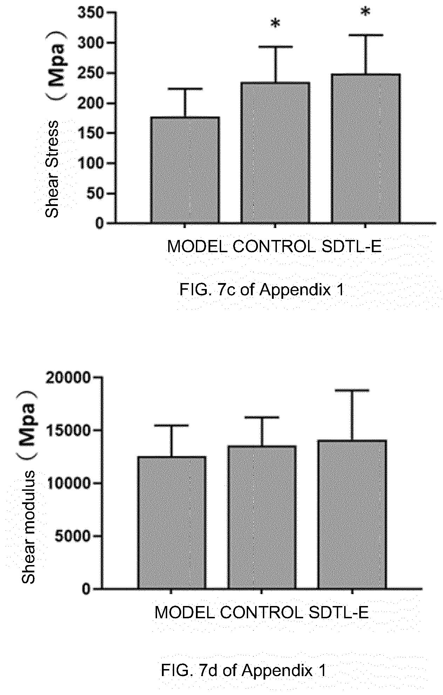

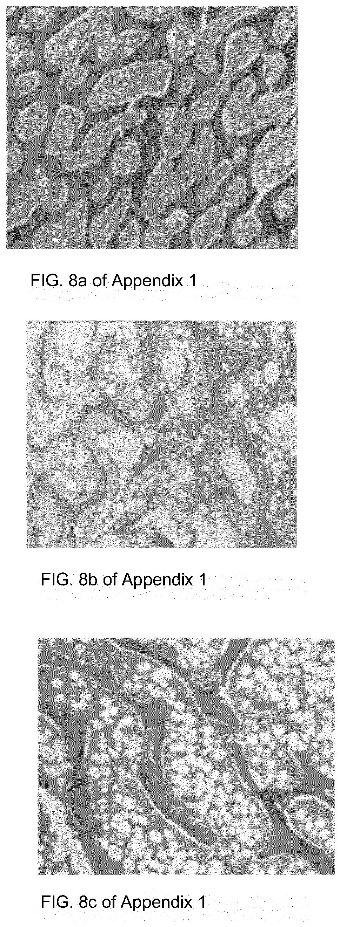

FIG. 4 shows the effect of INVENTED COMPOSITION on serum alkaline phosphatase in mice removed from ovarian models in animal experiments according to one embodiment.

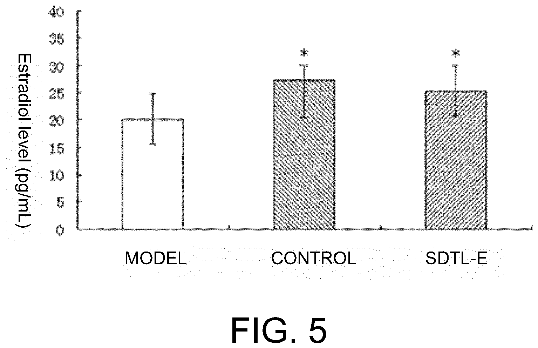

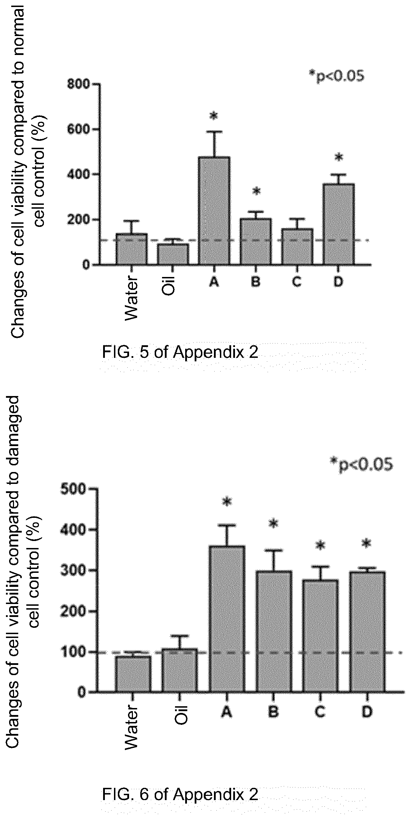

FIG. 5 shows the effect of INVENTED COMPOSITION on estradiol content in mice removed from ovarian models in animal experiments according to one embodiment.

### DETAILED DESCRIPTIONS

As a brief discussion, matters are composed of different molecules and molecular structures, and each substance has a different molecular formula, spatial structure and functional role. In all material space, the space inside and outside the matter or molecular cross space and the resulting functional role is a variable and specific relationship, and its specific amount because of other specific factors to participate in the variable change, and will produce different functional changes and results. In the same environmental space of the composite chemical composition of the type, quantity and mutual proportion is a specific dynamic and static balance relationship, and the mutual specific balance has an important impact on the function of the material and the disappearance thereof along with the strength of the function of the effect. The more the number of different substances and molecules in the same space, the more their molecular intersections and changes are identified in the specific amount. At the same time, the more the specific amount of change process in space, the more difficult it is to appreciate the specific amount of change. At the same time, a number of factors may affect its functional effect and utility. For example, one of the following may demonstrate the issues: the less the specific factors of matter and molecules in the same space, the less the molecular intersection and the specific amount of change, the less the process variable in the physical space, the less the degree of change and its specific result, whether the selection of the variety of the substance is appropriate and whether the proportion is suitable and the use (such as oral, injection, skin external use, inhalation, etc.). These may affect its functional effect and utility results, and natural plants may produce new drug treatment functions and effects through complex components and unknown biochemical reactions or electron displacement reactions. Similarly, different ways of use may also go beyond the researcher's efforts or expectations, and the combination of all these may bring unexpected results.

In nature, bones are one of the main organ tissues of vertebrate organisms. One of its functions is to support, protect the body and through joints and muscles, nerve tissues to maintain the ability of organisms to exercise. Bones have the function of storing minerals, mainly composed of bone, bone marrow and periosteum, bone joints (articulation) is the bone and bone may move and change the angle between the part, between which there are cartilage, joint capsules, ligaments and synovial fluid. All osteoarticular dysfunction diseases lead to a decrease or complete loss of the ability of organisms (including the human body) to move and move, and osteoporosis and degenerative arthritis (also known as degenerative arthritis) are one of the most important diseases in orthopedics and motor impairments in human, with a large number of patients worldwide. Joint degeneration and osteoporosis are more common in patients in developed and advanced countries and regions than in underdeveloped areas, and mostly exist in groups of people over 40 years old (especially in postmenopausal women), and the trend is developing toward young people is becoming more and more obvious. A few years ago, there were data showing that a few years ago, the world's degenerative arthritis patients alone have exceeded 800 million people and degenerative arthritis and osteoporosis diseases seriously affect people's ability to move and live independently. Modern medicine treatment of osteoarticular degeneration and osteoporosis is mainly surgery, injection and oral medication. To this day, very few drugs may effectively and quickly treat degenerative joints and osteoporosis and improve joint mobility. Topical natural medicines that are effective in treating degenerative joints and osteoporosis are almost non-existent.

In the field of disease treatment, topical drugs are generally administered through skin, the time of action of the drug and the effect of medicinal treatment are generally and significantly lower than the administration effect of oral and injectable mode. Aspects of the invention cover the use of selected natural plant extracts in an external method or topical method to use the biochemical diversity of natural plants to produce functional effects. In one embodiment, products embodying aspects of the invention may be applicable to youth, middle and old age degenerative when suffering from osteoarticular degeneration and osteoporosis disease and may improve symptoms thereof. In addition, aspects of the invention may be applicable to symptoms associated with arthritis of joint abnormalities, including joint weakness, muscle weakness, pain, squat difficulties, inability to go up and down stairs and other symptoms of the function. In addition, embodiments of the invention may improve osteoporosis estrogen reduction and hip pain. Further, aspects of the invention may be used topically to quickly reduce, eliminate and treat the clinical symptoms of osteoarticular degeneration and osteoporosis, applicable to regardless of age and ethnicity, sex, may also be applied to animals ( including but not limited to cats, dogs, etc.). As no oral and injection are required, the topical application of aspects of the invention make application simple, direct and easy to operate. Further, the extracts of aspects of the invention may be obtained by a variety of process methods, such as distillation, pressing and supercritical extraction. In another aspect, embodiments of the invention may be embodied in various products, such as daily chemical products, makeup, skin care, health care and other drugs, lotion dosage forms, and any other external use of the skin.

Degenerative arthritis and osteoporosis patients around the world are trending towards younger generations, and they are developing arthritis, joint abnormality, joint weakness, muscle weakness, pain, squat difficulties, unable to go up and down stairs and other inconvenient clinical symptoms and osteoporosis clinical symptoms-common in degenerative arthritis and osteoporosis patients and extremely difficult to improve and treat. To many patients, families and society are causing serious trouble and burden, and modern treatment methods existed today such as joint replacement surgery, joint hyaluronic acid injection, oral tablets of glucosamine, oral calcium supplementation and artificial chemotherapy (many with side effects) in addition to various treatment of degenerative arthritis and osteoporosis. There are no other treatment or improvement method with significant improvements. In one aspect, osteoporosis is a metabolic disease, and patients due to bone density reduction are therefore very prone to pain, fractures and complications. As such, calcium supplementation, vitamin D supplementation or exercise are one of the coping methods. However, these self-help effect is extremely limited, and the nonsteroidal antiinflammatory drug diclofenac (Diclofenac) is also used in the in vitro treatment/application of arthritis and osteoporosis. But due to the contraindication and side effects to pregnant women, the US Food and Drug Administration (FDA) pregnancy medication system classifies this drug as C moderate risk and D highly risky, with possible side effects include stomach upset, nausea and vomiting, heartburn, diarrhea, constipation, flatulence, headache, drowsiness, dizziness, etc., where these medication cannot be used in a prolonged fashion, may cause side effects, and long period of use.

Aspects of the invention, while with minimal side effects, may include an known but undesirable effect of light rash when applied on sweaty skin surface. This condition may be alleviated or eliminated in two days once the application is stopped.

Natural plants or extracts are one of the raw material sources of natural medicines for human beings, plants extracting single compounds (such as artemisinin, lutein, eleulin, etc.) have long been widely used in medicine, beauty and related industries or products, and widely used in anti-aging and natural medicine production. Plant extraction of a single compound in the extraction process may be remove harmful substances to the human body such as heavy metals, pesticide residues and other harmful substances. As such single compound medicines may provide humans with better and higher equivalent concentration of pharmaceutical raw material ingredients, which further highlights the function of a single compound as it is focused and the medicinal effect is specific while the target and pharmacology are clear. However, because of the unity of its chemical composition, it is also very easy for the human body to develop drug resistance.

Natural plants have better biological recognition and compatibility with organisms due to the large number of natural chemical components. After studying and analyzing as well as understanding and evaluating many plants by many methods, the following are screened from many plants: ginger (Zingiber cfflcinale), pepper (capsicum annuum), onion (allium cepa), garlic (allium sativum), pepper (piper nigrum), peppercorn (spicy), bergamot (citrus medica variable), lemon (citrus limon), orange (citrus reticulata), green olive (mayarium album) (fruit), rose (rosa rugosa), lavender (lavandula), vetiver Andropogon zizanioides, angelica (sinensis), citronella (cymbopogon (aromatic oil)), chrysanthemum (cymbopogon), camellia japonica, osmanthus fragrans (mosaic), black fungus or mushroom (augricularia auricula), white fungus or mushroom (tremella fuciformis), red algae (rhodophyta (containing colloids)), Korean ginseng (panax ginseng), North American ginseng (panax quinquefolius), ginseng (codonopsis pilosula), cucumber (cucumis sativus), pumpkin (cucurbita cv), loofah (luffa aegyptiaca (melon)), or the like. When providing each of the natural plants or extracts to a very small number of knee degeneration and osteoporosis patients during a trial, the trial patients' subjective comments, in one embodiment, found that vetiver vetiveria zizanioides and its cognate plants (e.g., those plants or grass in the species gramineae or poaceae), such as vetiver (chrysopogon zizanioides) or Andropogon zizanioides extract has a relieving effect on knee pain phenomena (less than about 2 hours) while providing an improvement on joint stiffness and mobility. In one embodiment, carrot seed extracts further include ameliorating effects on joint stiffness. In a further embodiment, vetiver (vetiveria zizanioides) and its cogeneration (such as plants or grass in the species gramineae or poaceae, including the vetiver chrysopogon zizanioides or Andropogon zizanioides) extract as the main ingredient substance of the invention and combine with extract of carrot or Daucus carota, along with other extracts as a combination, may provide better, faster, more pronounced functional effects and therapeutic effects.

As such, in one embodiment, aspects of the invention may include a composition having natural plant vetiveria zizanioides and its cogeneration (e.g., vetiver (chrysopogon zizanioides) or Andropogon zizanioides) or an extract thereof as a main ingredient for an external treatment applied on the skin and methods of improving joint degeneration and osteoporosis and alleviate stiffness in joints. In one embodiment, the extract may be dissolved with other substances (including but not limited to oils, extracts, solvents). In one embodiment, the extract and the solvent may be in a weight ratio range of 1 to 9 : 9 to 1. In another embodiment, another weight ratio ranges may be from 1 to 7:3 to 9. In yet another embodiment, another weight ratio ranges may be from 1 to 5:5 to 9.

In one embodiment, in another embodiment, products may be a natural plant vetiveria zizanioides and its cogeneration (e.g., vetiver (chrysopogon zizanioides)) or Andropogon zizanioides and their extracts. The product may be combined or mixed with carrot seed or carrot-containing extract to treat and improve joint degeneration and osteoporosis in a topical way to the skin. In one aspect, the product may be formulated with vetiver vetiveria zizanioides and those in the same species (such as vetiver) (chrysopogon zizanioides) or Andropogon zizanioides extract as the main ingredient. In another aspect, extracts of carrot or Daucus carota may be mixed. In yet another embodiment, the weight ratio of the 2 extracts to each other (e.g., vetiver and carrot seed) may range from about 1 to 9:2 to 9. In another embodiment, the proportional range may be about 1.5 to 7.5:2.5 to 8.5. In yet another embodiment, a proportion range may be about 2 to 5:4.5 to 8.

In one embodiment, the external use of the skin in the present invention may restore the ovary mouse estrogen and serum alkaline phosphatase content to normal levels, and these pathological changes of osteoporosis caused by estrogen deficiency have a therapeutic and protective effect, thus may contribute to osteoarticular degeneration and osteoporosis related disease research and treatment.

In one embodiment, the present invention of a natural substance skin external treatment of the disease method may treat human osteoporosis hip pain and joint degeneration of the hip ring, joint inability to bend, joint weakness, muscle weakness, squatting difficulties, difficulty going up and down stairs,.

In one embodiment, the extracts of the invention may include but are not limited to distillation, extraction, supercritical and other process methods obtained. In one aspect, the formulation may be recombined or dissolved with other substances, may be in different forms and dosage forms (including but not limited to liquids, sprays, smears, lotions, creams, etc.) use.

In one embodiment, aspect of the invention may improve the clinical symptoms of human degenerative arthritis and osteoporosis, improve joint mobility, increase joint range of motion and reduce joint pain. In another embodiment, aspects of the invention have the effect of improving joint from making abnormal sound, improving joint inability to bend, improving joint weakness, improving muscle weakness, improving squat difficulty, improving stairs difficulty and other clinical symptoms, and improving osteoporosis hip pain.

In one embodiment, the present invention may be applied to improve the animal osteoarticular degeneration and osteoporosis caused by discomfort phenomenon.

Aromatase (Aromatase) is known as estrogen synthetase (estrogen synthetase), which can catalyze the aromatization reaction from androgen to estrogen in steroid hormone synthesis process, catalyze androstenedione, testosterone to estrone And estradiol conversion, aromatase exists in gonads, brain, adipose tissue, blood vessels, skin and bones, and the rate of aromatase reaction can be regulated by coenzyme. The experimental results of SDTL-E, a product based on aspects of the invention, in osteoporosis mice suggest that SDTL-E (hereinafter as a short-name for products embedding aspects of the invention) may exhibit the effect of aromatization-accelerating coenzyme (Aromatization Acceleration Coenzyme), and the aromatization-accelerating coenzyme cluster produced by the compounding of its extracts [Aromatization Acceleration Coenzyme Cluster] has a regulatory effect on aromatase (Aromatase), and its mechanism may be through the activation of aromatase (Aromatase) enzymatic reaction (Enzyme catalysis) to make abnormal hormone levels, including estradiol normalization and abnormal ALP activity, serum calcium, urinary calcium, etc. to be returned to normal, which promoted the effect of osteoclasts and osteoblasts on the body, and showed the effect of drug therapy to restore abnormal hormones to normal levels and promote the recovery of bone strength.

**Embodiments:**

In the vetiver vetiveria zizanioides and their cogenerations (such as vetiver (chrysopogon zizanioides) or Andropogon zizanioides, or the extracts thereof may be combined with carrot extract to provide a strong or pronounced analgesic and synergistic effect, as well as may alleviate joint stiffness, joint abnormal sound, joint curvature and improve joint mobility. With the combined composition of vetiver vetiveria zizanioides and those in the same species (such as gramineae or poaceae), vetiver (chrysopogon zizanioides) or Andropogon zizanioides extracts and carrot (and its extracts of carrot or Daucus carota) as the main ingredient formulation (hereinafter referred to as "SDTL-E" or "invented composition"). In these 2 compositions, the vetiver vetiveria zizanioides may be the main natural constituent substance or the active ingredient. For example, the invented composition may include vetiver alcohol, benzoic acid, iso eugenol, vanillin, valeren, juniperene, limelan acid, vetiver alcohol, vetiverone and sesquiterpene, aldehydes, alcohols and other compound components. Carrot seed (including Daucus carota) ingredient may include monothenes, esters, monothetyls, oxides, ethers, carrots, and vitamins B, C, E, sesquiphenene, sesquiciferol and carotenesin, carotenes, no arrhophones, trans-β-acacia alkenes and other natural chemical ingredients. The invented composition mainly include these two natural extracts mixed in a desirable or proportional range for human and animal use. Further, according to animal experiments and human use statistics, embodiments of the invented composition may provide a strong practicality, obvious efficacy, rapid action, a wide range of effects and its ease to use or application with a number of advantages. Further, embodiments of the invented composition may combine with other extracts, compounds, chemicals, greases, solvents, water, emulsions and other substances. For example, embodiments of the invented composition may be in different forms, such as drops, sprays, smears, coatings and other ways to apply to the skin, may be used in animals and human bodies.

### Example 1:

Estrogen deficiency is one of the main causes of postmenopausal osteoporosis and joint degeneration, and in the small intestine, osteoblasts, osteoclast precursors, bone cells and growth bone plates and other cartilage cells have *estrogen* receptors, postmenopausal estrogen levels decreased and serum alkaline phosphatase content increase is one of the most important causes of joint degeneration and osteoporosis in women. Embodiments of the invented composition may be used in the ovariectomized (OVX) osteoporosis 040 of mouse skin to observe changes in mouse in vivo data. Final experiments showed that after treatment with mice on the skin, the serum alkaline phosphatase content in mice in ovary deprived or ovary-removed models was basically reduced and returned to near normal levels (P<0.01), and the estrogen in ovary deprived or ovary-removed or (OVX) models was basically increased to near normal levels (P<0.01) **(see Annex 1 Animal Experiments).**

In one embodiment, it is shown that the embodiments of invented composition may increase estrogen in biological mice and reduce the serum alkaline phosphatase content of mice to normal levels, have the effect of balancing estrogen and serum alkaline phosphatase content in ovary deprived or ovary-removed model rats, and have potential research and practical application value in the treatment of joint degeneration and osteoporosis. For example, in one embodiment, the selected de-ovulatory mouse experimental model is rich in literature, mature technology and stable and reliable, and is the best model for OVX mouse research recommended by the World Health Organization WHO and the National Institutes of Health (NIH).

In one embodiment, the embodiments of the invented composition formulation may be combined with the liquid extract or form of vetiver and the carrot Daucus carota as the main extract. In one embodiment, the two extracts of the aforementioned substances may be shaken alone or separately before combining and then stirred for less than 10 seconds. In one embodiment, the weight ratio of the two extracts or substances to each other ranges from about 1 to 9: 2 to 9. In one embodiment, a range from about 1.5 to 7.5: 2.5 to 8.5 may be used. In one embodiment, a range of about 2 to 5:4.5 to 8 may be used. In one embodiment, the two ingredients may be mixed at temperature about 4 ° C or above in an environment of >40 rpm / second for mixing or stirring for 5 seconds or more before letting it sit to settle (the time or mixing speed may vary or be adjusted depending on the actual proportion of the total weight). In another embodiment, in adding grease, the grease may dissolve in about 1 to 9 : 9 to 1 weight ratio range. In another embodiment, another weight ratio range may be 1 to 7 : 3 to 9. In yet another embodiment, a third weight ratio range may be about 1 to 5: 5 to 9. In external skin application (e.g., topical) on experiment animals, the results showed a decrease in the compensatory elevation of early alkaline phosphatase (ALP) on model mice, had an increasing effect on mouse estradiol hormone, and thus may reduce osteoporosis in osteoporosis animals, while having the therapeutic function and balance effect of restoring estrogen and serum alkaline phosphatase. Such result shows a protective and therapeutic effect on the pathological changes of osteoporosis caused by estrogen deficiency, and may be studied and developed into a topical drug for the treatment of osteoarticular degeneration and osteoporosis. **(See Annex 1: Animal Experimentation).**

### Example 2:

In one embodiment, the natural plant vetiveria zizanioides and those in the same species (e.g., vetiver (chrysopogon zizanioides) or Andropogon Zizanioides), the extracts thereof may be diluted with a solvent (alcohol, oil, etc.) and used in human degenerative arthritis knee skin. In one embodiment, the extract and the solvent may be in the weight ratio range of about 1 to 9 : 9 to 1. In one embodiment, another weight ratio ranges from about 1 to 7: 3 to 9 may be used. In yet another embodiment, another weight ratio ranges from 1 to 5: 5 to 9 may be used. Aspects of the invention may provide the effect of alleviating and improving joint pain, stiffness, or the like. In one embodiment, when used in human degenerative arthritis knee skin twice a day, each time at 0.4 ml to eight patients, four patients reported a decreasing trend of joint pain and stiffness symptoms in the two hours immediately after use (trend rate of 50%). In another embodiment, it has been shown that a single extract of vetiver on joint degeneration pain, stiffness symptoms have an improving effect. In yet another aspect, after a variety of try-out and error, analysis and consideration, the invented composition formula with vetiver and carrot seed as a human skin topical reagent exhibit most noticeable effect from the statistics and patient reports when used on the skins of joint degeneration patients (voluntary users).

In one embodiment, aspects of the invented composition formulation by the vetiver vetiveria zizanioides and those in the same species (e.g., vetiver (chrysopogon zizanioides) or Andropogon zizanioides, carrot seed or extracts containing carrot glycosides may be formed. In one embodiment, the two extracts of the aforementioned substances may be shaken alone before combining and then stirred for greater than 10 seconds. In one embodiment, the weight ratio of the two extracts or substances to each other ranges from about 1 to 9: 2 to 9. In one embodiment, another weight ratio ranges from about 1.5 to 7.5: 2.5 to 8.5 may be used. In yet another embodiment, another weight ratio ranges from 2 to 5: 4.5 to 8 may be used. In one embodiment, the two ingredients may be mixed at temperature about 4 ° C or above in an environment of >40 rpm / second for mixing or stirring for 5 seconds or more before letting it sit to settle (the time or mixing speed may vary or be adjusted depending on the actual proportion of the total weight). In another embodiment, in adding grease, the grease may dissolve in about 1 to 9 : 9 to 1 weight ratio range. In another embodiment, another weight ratio range may be 1 to 7 : 3 to 9. In yet another embodiment, a third weight ratio range may be about 1 to 5: 5 to 9. In one embodiment, aspects of the invented composition were used in an experiment of 23 patients. In one example, the experiment of the 23 patients who are suffering from knee disorders and being aged among 50-96 years of trial, in one embodiment, involves subjecting to these 23 patients the invented composition two times a day, at 0.4 ml each time. After 48 hours of records, 21 patients show significant improvements out of 23 patients (with two being invalid). In such trial, the effective rate of aspects of the invented composition was 92.93%. In another embodiment, the invented composition formula and topical application appears to show a significant reduction of clinical symptoms of human degenerative arthritis while increasing ranges of joint movement range, improving joint mobility and walking and motor capacity of therapeutic effect (see Table 1 for details).

Another 17 people aged among 55-81 years with osteoporosis hip pain were tested once a day for 0.4 ml, with records at 20 minutes and 48 hours after application. Among the 17 people, 16 people had significant improvement in symptoms (1 person was ineffective), and the statistical effectiveness rate was 94.118%. (See Table 3).

**Table 1:**

| Example 2 | | | | |
|---|---|---|---|---|
| Degenerative arthritis skin topical application of the INVENTED COMPOSITION 0.4 ml twice a day. | | | | |
| Statistical table of effects, the number of statistics is 23 (11 males, 12 females, age 50-96 years). | | | | |
| Clinical symptoms | Number of users | Use 1 time Number of valid people (within 20 minutes). | Use 4 times having significant number of valid people (48 hours). | Average efficiency |
| Joint noise | 23 | 21 | 21 | 91.3% |
| Joint weakness | 23 | 22 | 22 | 95.65% |
| Joint stiffness | 23 | 21 | 23 | 95.65% |
| Joint pain | 23 | 22 | 22 | 95.65% |
| Muscle weakness | 23 | 21 | 21 | 91.3% |
| Squatting is difficult | 23 | 21 | 21 | 91.3% |
| Difficulty going up and down the stairs | 23 | 21 | 21 | 91.3% |
| Range of motion of the joints | 23 | 21 | 21 | 91.3% |
| Average total effective rate | | | | 92.93% |

Degenerative arthritis skin topical application of the INVENTED COMPOSITION applied twice daily at 0.4 ml.

Approximately 20 minutes, 48 hours questionnaire, 23 people (11 males, 12 females, age 50-96 years).

**Table 2:**

| serial numb er | gen der age | Time | Rang e of motio n of the joints (degr ee of impro veme nt). | joint Abno rmal soun d (degr ee of impro veme nt). | joint Wea kne ss (deg ree of impr ove men t). | mus cles Wea kne ss (deg ree of impr ove men t). | Squ at Diffi cult y (deg ree of impr ove men t). | Up and dow n stair cas e Diffi cult y (deg ree of improve men t). | Join t pain (De gree of impr ove men t). | Join t stiff nes s (deg ree of impr ove men t). |
|---|---|---|---|---|---|---|---|---|---|---|
| 1 | wo ma n 56 | 20 points | impro ve | impro ve | impr ove | impr ove | impr ove | impr ove | impr ove | impr ove |
| | | 48 hours | obvio us | obvio us | obvi ous | obvi ous | obvi ous | obvi ous | obvi ous | obvi ous |
| 2 | wo ma n 50 | 20 minutes | impro ve | impro ve | impr ove | impr ove | impr ove | impr ove | impr ove | impr ove |
| | | 48 hours | obvio us | obvio us | obvi ous | obvi ous | obvi ous | obvi ous | obvi ous | obvi ous |
| 3 | ma n 62 | 20 minutes | impro ve | impro ve | impr ove | impr ove | impr ove | impr ove | impr ove | impr ove |
| | | 48 hours | obvio us | obvio us | obvi ous | obvi ous | obvi ous | obvi ous | obvi ous | obvi ous |
| 4 | wo ma n 96 | 20 minutes | impro ve | impro ve | impr ove | impr ove | impr ove | impr ove | impr ove | impr ove |
| | | 48 hours | obvio us | obvio us | obvi ous | obvi ous | obvi ous | obvi ous | obvi ous | obvi ous |
| 5 | ma n 71 | 20 minutes | impro ve | impro ve | impr ove | impr ove | impr ove | impr ove | impr ove | impr ove |
| | | 48 hours | obvio us | obvio us | obvi ous | obvi ous | obvi ous | obvi ous | obvi ous | obvi ous |
| 6 | wo ma n 65 | 20 minutes | void | void | impr ove | void | void | void | impr ove | void |
| | | 48 hours | void | void | impr ove | void | void | void | impr ove | void |
| 7 | wo ma n 67 | 20 minutes | impro ve | impro ve | impr ove | impr ove | impr ove | impr ove | impr ove | impr ove |
| | | 48 hours | obvio us | obvio us | obvi ous | obvi ous | obvi ous | obvi ous | obvi ous | obvi ous |
| 8 | ma n 81 | 20 minutes | impro ve | impro ve | impr ove | impr ove | impr ove | impr ove | impr ove | impr ove |
| | | 48 hours | obvio us | obvio us | obvi ous | obvi ous | obvi ous | obvi ous | obvi ous | obvi ous |
| 9 | ma n 85 | 20 minutes | impro ve | impro ve | impr ove | impr ove | impr ove | impr ove | impr ove | impr ove |
| | | 48 hours | obvio us | obvio us | obvi ous | obvi ous | obvi ous | obvi ous | obvi ous | obvi ous |
| 10 | wo ma n 55 | 20 minutes | impro ve | impro ve | impr ove | impr ove | impr ove | impr ove | impr ove | impr ove |
| | | 48 hours | obvio us | obvio us | obvi ous | obvi ous | obvi ous | obvi ous | obvi ous | obvi ous |
| 11 | ma n 76 | 20 minutes | impro ve | impro ve | impr ove | impr ove | impr ove | impr ove | impr ove | impr ove |
| | | 48 hours | obvio us | obvio us | obvi ous | obvi ous | obvi ous | obvi ous | obvi ous | obvi ous |
| 12 | wo ma n 58 | 20 minutes | impro ve | impro ve | impr ove | impr ove | impr ove | impr ove | impr ove | impr ove |
| | | 48 hours | obvio us | obvio us | obvi ous | obvi ous | obvi ous | obvi ous | obvi ous | obvi ous |
| 13 | wo ma n 69 | 20 minutes | impro ve | impro ve | impr ove | impr ove | impr ove | impr ove | impr ove | impr ove |
| | | 48 hours | obvio us | obvio us | obvi ous | obvi ous | obvi ous | obvi ous | obvi ous | obvi ous |
| 14 | ma n 73 | 20 minutes | impro ve | impro ve | impr ove | impr ove | impr ove | impr ove | impr ove | impr ove |
| | | 48 hours | obvio us | obvio us | obvi ous | obvi ous | obvi ous | obvi ous | obvi ous | obvi ous |
| 15 | wo ma n 57 | 20 minutes | impro ve | impro ve | impr ove | impr ove | impr ove | impr ove | impr ove | impr ove |
| | | 48 hours | obvio us | obvio us | obvi ous | obvi ous | obvi ous | obvi ous | obvi ous | obvi ous |
| 16 | ma n 71 | 20 minutes | impro ve | impro ve | impr ove | impr ove | impr ove | impr ove | impr ove | impr ove |
| | | 48 hours | obvio us | obvio us | obvi ous | obvi ous | obvi ous | obvi ous | obvi ous | obvi ous |
| 17 | ma n 67 | 20 minutes | impro ve | impro ve | impr ove | impr ove | impr ove | impr ove | impr ove | impr ove |
| | | 48 hours | obvio us | obvio us | obvi ous | obvi ous | obvi ous | obvi ous | obvi ous | obvi ous |
| 18 | ma n 76 | 20 minutes | impro ve | impro ve | impr ove | impr ove | impr ove | impr ove | impr ove | impr ove |
| | | 48 hours | obvio us | obvio us | obvi ous | obvi ous | obvi ous | obvi ous | obvi ous | obvi ous |
| 19 | wo ma n 58 | 20 minutes | impro ve | impro ve | impr ove | impr ove | impr ove | impr ove | impr ove | impr ove |
| | | 48 hours | obvio us | obvio us | obvi ous | obvi ous | obvi ous | obvi ous | obvi ous | obvi ous |
| 20 | wo ma n 67 | 20 minutes | void | void | void | void | void | void | void | void |
| | | 48 hours | void | void | void | void | void | void | void | void |
| 21 | ma n 87 | 20 minutes | impro ve | impro ve | impr ove | impr ove | impr ove | impr ove | impr ove | impr ove |
| | | 48 hours | obvio us | obvio us | obvi ous | obvi ous | obvi ous | obvi ous | obvi ous | obvi ous |
| 22 | ma n 79 | 20 minutes | impro ve | impro ve | impr ove | impr ove | impr ove | impr ove | impr ove | impr ove |
| | | 48 hours | obvio us | obvio us | obvi ous | obvi ous | obvi ous | obvi ous | obvi ous | obvi ous |
| 23 | wo ma n 61 | 20 minutes | impro ve | impro ve | impr ove | impr ove | impr ove | impr ove | impr ove | impr ove |
| | | 48 hours | obvio us | obvio us | obvi ous | obvi ous | obvi ous | obvi ous | obvi ous | obvi ous |

Note: The invalid or ineffective result may indicate that the patient is not a patient with osteoporosis but other inflammation.

**Table 3:**

| Patients with osteoporosis hip pain after embodiments of the invented composition applied through a topical application at 0.4 ml once a day | | | | |
|---|---|---|---|---|
| Statistical table of 48-hour effects, the total number of statistics is 17 (4 males, 13 females, age 55-81 years). | | | | |
| **Clinical symptoms** | **Number of trials** | **20 minutes Improve the number of people** | **48 hours Improve the number of people** | **Efficient** |
| Hip pain | 17 | 16 | 16 | 94.118% |
| **Total efficiency** | | | | **94.118%** |

Osteoporosis hip pain 0.4 m I twice daily, invented composition skin topical.

48-hour effect questionnaire, the number of people counted 17 (4 males, 13 females, 55-81 years old).

| **serial number** | **gender** | **age** | **Symptoms** | **20 minutes Improve pain** | **48 hours Improve pain** |
|---|---|---|---|---|---|
| 1 | woman | 59 | Hip pain | obvious | obvious |
| 2 | woman | 62 | Hip pain | obvious | obvious |
| 3 | woman | 65 | Hip pain | obvious | obvious |
| 4 | woman | 67 | Hip pain | obvious | obvious |
| 5 | man | 81 | Hip pain | Not obvious and invalid | Not obvious and invalid |
| 6 | woman | 66 | Hip pain | obvious | obvious |
| 7 | woman | 67 | Hip pain | obvious | obvious |
| 8 | man | 76 | Hip pain | obvious | obvious |
| 9 | man | 73 | Hip pain | obvious | obvious |
| 10 | woman | 55 | Hip pain | obvious | obvious |
| 11 | man | 72 | Hip pain | obvious | obvious |
| 12 | woman | 58 | Hip pain | obvious | obvious |
| 13 | woman | 69 | Hip pain | obvious | obvious |
| 14 | woman | 73 | Hip pain | obvious | obvious |
| 15 | woman | 57 | Hip pain | obvious | obvious |
| 16 | woman | 71 | Hip pain | obvious | obvious |
| 17 | woman | 67 | Hip pain | obvious | obvious |

| | | | | | |
|---|---|---|---|---|---|
| Note: The invalid or ineffective result may indicate that the patient is not a patient with osteoporosis but other inflammation. | | | | | |

In one embodiment, human trials show a single ingredient of vetiver vetiveria zizanioides and those in the same species (e.g., vetiver (chrysopogon zizanioides) or Andropogon Zizanioides) or their extracts have the effect of improving joint pain and stiffness. In one embodiment, aspects of the invented composition provide a better effectiveness than a single vetiver extract therapeutic effect as effects are realized faster. In one embodiment, also confirmed by animal experiments, embodiments of the invented composition formula may boost estrogen in de-ovulatory mice, reduce serum alkaline phosphatase content or the like. Several cases of extra-dermal or topical use in cats or dogs have been observed, which also show an improving effect on joint movement disorders. These examples may be applied to other animal therapeutic uses, and the formula may be made into different dosage forms including but not limited to creams.

In addition to the above experimental data, Appendices 2 to 4 provide other experimental reports that support the characteristics of embodiments of the present invention, including the impact and comparison of SDTL-E on cell viability; the impact of SDTL-E on intracellular reactive oxygen species; and combinations of different proportions of SDTL-E Effects of substances on the activity of cellular potassium channels.

Although variable and depending on the application, but is used in examples of methods or components disclosed herein. Those familiar with this technique will realize that some steps may be performed in any actual order to make an ingredient or compound for the desired use.

The above description and schema only explain and illustrate the present invention, the present invention is not limited thereto. Although the specification is described with respect to certain embodiments or embodiments, but for illustrative purposes elaborate many details. Accordingly, the above content only illustrates the principles of the invention. For example, the present invention may have other specific forms without departing from its spiritual or essential features. The layout described is illustrative rather than restrictive. For those familiar with this technique, the present invention allows additional embodiments or embodiments, and some of these details described in the present application may vary considerably without departing from the basic principles of the invention. It should be appreciated that those familiar with this technique may design various schemes, although not explicitly described or shown herein, but embody the principles of the invention, and therefore within its scope and spirit.

The above instructions are illustrative and not restrictive. When reviewing disclosures, many changes in the embodiment may become apparent. Therefore, the scope of the embodiment should not be determined by reference to the above statement, but should be determined by reference to the pending claim of rights and all of its scope or equivalent.

One or more features in any embodiment may be used in conjunction with one or more features of any other embodiment, without deviating from the scope of the embodiment. For "one", "single", or "some" is intended to mean "one or more", unless specifically noted to the contrary. The recitation of "and/or" is intended to represent the most inclusive consciousness in the term, unless explicitly stated to the contrary.

While this disclosure may be embodied in many different forms, the understanding at the time of submission and discussion is that this disclosure is the embodiment of one or more of the principles of invention and is not intended to limit any one embodiment to the embodiment described.

This disclosure provides a solution to these long-term needs. In particular, aspects of the invention overcome the reliance on existing mixtures using unnatural or chemical formulations to treat and improve joint degeneration and osteoporosis, help restore mobility and eliminate pain.

Further advantages and modifications of the above ingredients and methods, may easily occur in those skilled in the art.

Therefore, disclosure is not limited to specific details, representative systems and methodologies, and the illustrative examples described above. Various modifications and modifications may be made to the above specifications without departing from the scope or spirit of this disclosure, which shall cover all such modifications and changes, provided that such modifications and modifications fall within the scope of the following claims and their equivalents.

## Claims

1. A skin topical composition comprising a first active ingredient and a second active ingredient, wherein the first active ingredient comprises a first plant in a species of Gramineae or poaceae, wherein the first plant comprises vetiveria zizanoides, vertiver, chrysopogon zizanioides, or Andropogon zizanioides.

2. The skin topical composition of claim 1, wherein the second active ingredient comprises a second plant in a species of Apiaceae, wherein the second plant comprises Daucus carota.

3. The skin topical composition of claim 1, wherein first active ingredient or the second active ingredient comprises an extract of the first plant or the second plant.

4. The skin topical composition of claim 1, wherein the first and second active ingredients are used to improve osteoporosis, joint degeneration, joint stiffness, joint mobility, and physical reactions caused.

5. The skin topical composition of claim 1, wherein the first and second active ingredients are used to improve bone strength.

6. The skin topical composition of claim 1, wherein the first or second active ingredient regulates an abnormal level of hormones and improves discomfort caused by the abnormal level.

7. A skin topical composition comprising a first active ingredient, wherein the first active ingredient comprises a first plant in a species of Gramineae or poaceae, wherein the first plant comprises vetiveria zizanoides, vertiver, chrysopogon zizanioides, or Andropogon zizanioides.

8. The skin topical composition of claim 7, wherein the first active ingredient comprises an extract of the first plant.

9. The skin topical composition of claim 7, wherein the first active ingredients is used to improve osteoporosis, joint degeneration, joint stiffness, joint mobility, and physical reactions caused.

10. The skin topical composition of claim 7, wherein the first active ingredients is used to improve bone strength.

11. The skin topical composition of claim 7, wherein the first active ingredient regulates an abnormal level of hormones and improves discomfort caused by the abnormal level.

12. A skin topical composition comprising a first active ingredient, wherein the first active ingredient comprises a first plant in a species of Apiaceae, wherein the second plant comprises Daucus carota.

13. The skin topical composition of claim 12, wherein the first active ingredient comprises an extract of the first plant.

14. The skin topical composition of claim 12, wherein the first active ingredients is used to improve osteoporosis, joint degeneration, joint stiffness, joint mobility, and physical reactions caused.

15. The skin topical composition of claim 12, wherein the first active ingredients is used to improve bone strength.

16. The skin topical composition of claim 12, wherein the first active ingredient regulates an abnormal level of hormones and improves discomfort caused by the abnormal level.

17. A composition for regulation a balance level of hormones comprising a mixture, wherein the mixture comprises a first active ingredient and a second active ingredient, wherein the mixture is applied externally to a skin surface, wherein the first active ingredient comprises a first plant in a species of Gramineae or poaceae, wherein the first plant comprises vetiveria zizanoides, vertiver, chrysopogon zizanioides, or Andropogon zizanioides, wherein the second active ingredient comprises a second plant in a species of Apiaceae, wherein the second plant comprises Daucus carota.

18. The composition of claim 17, wherein the first active ingredient to the second active ingredient is at a ratio of 1 to 9 : 2 to 9 by weight.

19. The composition of claim 17, wherein the first active ingredient to the second active ingredient is at a ratio of 1.5 to 7.5 : 2.5 to 8.5 by weight.

20. The composition of claim 17, wherein the first active ingredient to the second active ingredient is at a ratio of 2 to 5 : 4.5 to 8 by weight.

21. The composition of claim 17, further comprising a solvent medium.

22. The composition of claim 21, wherein the solvent medium comprises a lipid solvent.

23. The composition of claim 21, wherein the mixture to the solvent medium is at a ratio of 1 to 9 : 9 to 1 by weight.

24. The composition of claim 21, wherein the mixture to the solvent medium is at a ratio of 1 to 7 : 3 to 9 by weight.

25. The composition of claim 21, wherein the mixture to the solvent medium is at a ratio of 1 to 5 : 5 to 9 by weight.

26. The composition of claim 17, wherein the first active ingredient comprises an extract of the first plant.

27. The composition of claim 17, wherein the second active ingredient comprises an extract of the second plant.

28. The composition of claim 17, wherein the first and second active ingredients are used to improve osteoporosis, joint degeneration, joint stiffness, joint mobility, and physical reactions caused.

29. The composition of claim 17, wherein the first and second active ingredients improve bone strength.

30. The composition of claim 17, wherein the first and second active ingredients regulate an abnormal level of hormones and improves discomfort caused by the abnormal level.
